(19)

**Europäisches Patentamt**
**European Patent Office**
**Office européen des brevets**

(11) **EP 3 868 293 B1**

(12) **EUROPEAN PATENT SPECIFICATION**

(45) Date of publication and mention
of the grant of the patent:
**29.03.2023 Bulletin 2023/13**

(21) Application number: **21168451.9**

(22) Date of filing: **17.09.2019**

(51) International Patent Classification (IPC):
*A61B 7/00* *(2006.01)*          *A61B 5/08* *(2006.01)*
*A61B 5/113* *(2006.01)*         *A61B 5/00* *(2006.01)*
*A61B 5/0205* *(2006.01)*

(52) Cooperative Patent Classification (CPC):
**A61B 7/003; A61B 5/0803; A61B 5/0816;**
**A61B 5/113; A61B 5/6898; A61B 5/7275;**
A61B 5/0002; A61B 5/0205; A61B 5/7257;
A61B 5/7264; A61B 5/7282; A61B 5/7285

(54) **SYSTEM AND METHOD FOR MONITORING PATHOLOGICAL BREATHING PATTERNS**

SYSTEM UND VERFAHREN ZUR ÜBERWACHUNG PATHOLOGISCHER ATMUNGSMUSTER

SYSTÈME ET PROCÉDÉ DE SURVEILLANCE DE SCHÉMAS RESPIRATOIRES PATHOLOGIQUES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **20.09.2018 US 201862733832 P**
**01.03.2019 US 201916290555**

(43) Date of publication of application:
**25.08.2021 Bulletin 2021/34**

(62) Document number(s) of the earlier application(s) in
accordance with Art. 76 EPC:
**19197860.0 / 3 639 748**

(73) Proprietor: **Samsung Electronics Co., Ltd.**
**Suwon-si 16677 (KR)**

(72) Inventors:
• **RAHMAN, Md Mahbubur**
**16677 Suwon-si (KR)**
• **CHATTERJEE, Soujanya**
**16677 Suwon-si (KR)**
• **NEMATI, Ebrahim**
**16677 Suwon-si (KR)**
• **KUANG, Jilong**
**16677 Suwon-si (KR)**
• **GAO, Jun**
**16677 Suwon-si (KR)**

(74) Representative: **HGF**
**HGF Limited**
**1 City Walk**
**Leeds LS11 9DX (GB)**

(56) References cited:
**EP-A1- 3 282 950     US-A1- 2015 313 484**
**US-B1- 9 610 042**

**Description**

**TECHNICAL FIELD**

This disclosure relates generally to pulmonary condition monitoring systems and methods. More specifically, this disclosure relates to a system and method for monitoring pathological breathing patterns using a mobile device.

**BACKGROUND**

Chronic respiratory diseases (chronic diseases of the airways) currently affect an estimated 40 million people in the United States alone. Common respiratory diseases include asthma, chronic obstructive pulmonary disease (COPD), occupational lung disease, and chronic bronchitis. More than half of those who suffer from respiratory diseases experience exacerbations or attacks related to those diseases every year. More than $130 billion in health care costs are associated with asthma and COPD annually, and pulmonary diseases are the third leading cause of death worldwide. Treatments for pulmonary diseases are often reactive in nature, meaning a patient is treated by a doctor or medication after an exacerbation or attack has already been triggered.

EP 3282950 A1 discusses a device, a system and a method for detecting a cardia and/or respiratory disease of a subject. EP 3282950 discloses the combination of features defined in the preambles of independent claims 1 and 6. US 9610042 B1 discusses systems and methods for detecting a medical condition using a mobile computing device. US 2015/313484 A1 discusses a portable device with multiple integrated sensors for vital signs scanning.

**SUMMARY**

This disclosure provides a system and method for monitoring pathological breathing patterns.

A first embodiment of the claimed invention is a computer program adapted to perform a method for pulmonary condition monitoring comprises: obtaining motion data collected by one or more other electronic devices; obtaining audio data received by a separate microphone; identifying respiratory phases of the user's breathing based on the audio data; producing breathing waveform data based on the motion data; converting the audio data into image data; identifying an abnormal sound associated with the user's breathing based on the image data; and identifying a pulmonary condition of the user based on the abnormal sound, the identified respiratory phases and the breathing waveform data.

A second embodiment of the claimed invention is an electronic device includes at least one memory and at least one processor coupled to the at least one memory. The at least one processor is configured to: obtain motion data collected by one or more other electronic devices; obtain audio data received by a separate microphone; identify respiratory phases of the user's breathing based on the audio data; produce breathing waveform data based on the motion data; convert the audio data into image data; identify an abnormal sound associated with the user's breathing based on the image data; and identify a pulmonary condition of the user based on the abnormal sound, the identified respiratory phases and the breathing waveform data.

In a third embodiment, a computer readable medium contains a program according to the first embodiment.

Before undertaking the DETAILED DESCRIPTION below, it may be advantageous to set forth definitions of certain words and phrases used throughout this patent document. The terms "transmit," "receive," and "communicate," as well as derivatives thereof, encompass both direct and indirect communication. The terms "include" and "comprise," as well as derivatives thereof, mean inclusion without limitation. The term "or" is inclusive, meaning and/or. The phrase "associated with," as well as derivatives thereof, means to include, be included within, interconnect with, contain, be contained within, connect to or with, couple to or with, be communicable with, cooperate with, interleave, juxtapose, be proximate to, be bound to or with, have, have a property of, have a relationship to or with, or the like.

Moreover, various functions described below can be implemented or supported by one or more computer programs, each of which is formed from computer readable program code and embodied in a computer readable medium. The terms "application" and "program" refer to one or more computer programs, software components, sets of instructions, procedures, functions, objects, classes, instances, related data, or a portion thereof adapted for implementation in a suitable computer readable program code. The phrase "computer readable program code" includes any type of computer code, including source code, object code, and executable code. The phrase "computer readable medium" includes any type of medium capable of being accessed by a computer, such as read only memory (ROM), random access memory (RAM), a hard disk drive, a compact disc (CD), a digital video disc (DVD), or any other type of memory. A "non-transitory" computer readable medium excludes wired, wireless, optical, or other communication links that transport transitory electrical or other signals. A non-transitory computer readable medium includes media where data can be permanently stored and media where data can be stored and later overwritten, such as a rewritable optical disc or an erasable memory device.

As used here, terms and phrases such as "have," "may have," "include," or "may include" a feature (like a number, function, operation, or component such as a part) indicate the existence of the feature and do not exclude the existence of other features. Also, as used here, the phrases "A or B," "at least one of A and/or B," or "one or more of A and/or B" may include all possible combinations of A and B. For example, "A or B," "at least one of A and B," and "at least one of A or B" may indicate all of (1) including at least one A, (2) including at least one B, or (3) including at least one A and at least one B.

As used here, the terms "first" and "second" may modify various components regardless of importance and do not limit the components. These terms are only used to distinguish one component from another. For example, a first user device and a second user device may indicate different user devices from each other, regardless of the order or importance of the devices. A first component may be denoted a second component and vice versa without departing from the scope of this disclosure.

It will be understood that, when an element (such as a first element) is referred to as being (operatively or communicatively) "coupled with/to" or "connected with/to" another element (such as a second element), it can be coupled or connected with/to the other element directly or via a third element. In contrast, it will be understood that, when an element (such as a first element) is referred to as being "directly coupled with/to" or "directly connected with/to" another element (such as a second element), no other element (such as a third element) intervenes between the element and the other element.

As used here, the phrase "configured (or set) to" may be interchangeably used with the phrases "suitable for," "having the capacity to," "designed to," "adapted to," "made to," or "capable of" depending on the circumstances. The phrase "configured (or set) to" does not essentially mean "specifically designed in hardware to." Rather, the phrase "configured to" may mean that a device can perform an operation together with another device or parts. For example, the phrase "processor configured (or set) to perform A, B, and C" may mean a generic-purpose processor (such as a CPU or application processor) that may perform the operations by executing one or more software programs stored in a memory device or a dedicated processor (such as an embedded processor) for performing the operations.

The terms and phrases as used here are provided merely to describe some embodiments of this disclosure but not to limit the scope of other embodiments of this disclosure. It is to be understood that the singular forms "a," "an," and "the" include plural references unless the context clearly dictates otherwise. All terms and phrases, including technical and scientific terms and phrases, used here have the same meanings as commonly understood by one of ordinary skill in the art to which the embodiments of this disclosure belong. It will be further understood that terms and phrases, such as those defined in commonly-used dictionaries, should be interpreted as having a meaning that is consistent with their meaning in the context of the relevant art and will not be interpreted in an idealized or overly formal sense unless expressly so defined here. In some cases, the terms and phrases defined here may be interpreted to exclude embodiments of this disclosure.

Examples of an "electronic device" according to embodiments of this disclosure may include at least one of a smartphone, a tablet personal computer (PC), a mobile phone, a video phone, an e-book reader, a desktop PC, a laptop computer, a netbook computer, a workstation, a personal digital assistant (PDA), a portable multimedia player (PMP), an MP3 player, a mobile medical device, a camera, or a wearable device (such as smart glasses, a head-mounted device (HMD), electronic clothes, an electronic bracelet, an electronic necklace, an electronic appcessory, an electronic tattoo, a smart mirror, or a smart watch). Other examples of an electronic device include a smart home appliance. Examples of the smart home appliance may include at least one of a television, a digital video disc (DVD) player, an audio player, a refrigerator, an air conditioner, a cleaner, an oven, a microwave oven, a washer, a drier, an air cleaner, a set-top box, a home automation control panel, a security control panel, a TV box (such SAMSUNG HOMESYNC, APPLETV, or GOOGLE TV), a gaming console (such as an XBOX, PLAYSTATION, or NINTENDO), an electronic dictionary, an electronic key, a camcorder, or an electronic picture frame. Still other examples of an electronic device include at least one of various medical devices (such as diverse portable medical measuring devices (like a blood sugar measuring device, a heartbeat measuring device, or a body temperature measuring device), a magnetic resource angiography (MRA) device, a magnetic resource imaging (MRI) device, a computed tomography (CT) device, an imaging device, or an ultrasonic device), a navigation device, a global positioning system (GPS) receiver, an event data recorder (EDR), a flight data recorder (FDR), an automotive infotainment device, a sailing electronic device (such as a sailing navigation device or a gyro compass), avionics, security devices, vehicular head units, industrial or home robots, automatic teller machines (ATMs), point of sales (POS) devices, or Internet of Things (IoT) devices (such as a bulb, various sensors, electric or gas meter, sprinkler, fire alarm, thermostat, street light, toaster, fitness equipment, hot water tank, heater, or boiler). Other examples of an electronic device include at least one part of a piece of furniture or building/structure, an electronic board, an electronic signature receiving device, a projector, or various measurement devices (such as devices for measuring water, electricity, gas, or electromagnetic waves). Note that, according to embodiments of this disclosure, an electronic device may be one or a combination of the above-listed devices. According to some embodiments of this disclosure, the electronic device may be a flexible electronic device. The electronic device disclosed here is not limited to the above-listed devices and may include new electronic devices depending on the development of technology.

In the following description, electronic devices are described with reference to the accompanying drawings, according to embodiments of this disclosure. As used here, the term "user" may denote a human or another device (such as an artificial intelligent electronic device) using the electronic device.

Definitions for other certain words and phrases may be provided throughout this patent document. Those of ordinary skill in the art should understand that in many if not most instances, such definitions apply to prior as well as future uses of such defined words and phrases.

The scope of patented subject matter is defined only by the claims.

## BRIEF DESCRIPTION OF THE DRAWINGS

For a more complete understanding of this disclosure and its advantages, reference is now made to the following description, taken in conjunction with the accompanying drawings, in which:

FIGURE 1 illustrates an example network configuration according to embodiments of this disclosure;
FIGURE 2 illustrates an example electronic device according to embodiments of this disclosure;
FIGURE 3 illustrates examples of breathing waveforms for different types of pulmonary conditions according to embodiments of this disclosure;
FIGURES 4A and 4B illustrate an example respiratory phase analysis of audio breathing data according to embodiments of this disclosure;
FIGURES 5A, 5B, and 5C illustrate example types of audio breathing data in a time domain and in a frequency domain according to embodiments of this disclosure;
FIGURE 6 illustrates an example architecture for wheeze detection using audio breathing data according to embodiments of this disclosure;
FIGURE 7 illustrates an example spectrogram of a severe wheezing episode as detected in a frequency domain chart according to embodiments of this disclosure;
FIGURE 8 illustrates an example severity chart for an asthma patient according to embodiments of this disclosure;
FIGURE 9 illustrates example breathing waveform data and respiratory phase information from at least one motion sensor according to embodiments of this disclosure;
FIGURE 10 illustrates example audio data and motion data collected from different sensors according to embodiments of this disclosure;
FIGURE 11 illustrates an example architecture for determining a pulmonary condition of a user and a severity of the pulmonary condition based on audio data and motion data according to embodiments of this disclosure;
FIGURE 12 illustrates an example process of predicting a user's pulmonary condition and severity using baseline data according to embodiments of this disclosure; and
FIGURE 13 illustrates an example method of issuing an alert based on collection of pulmonary data according to embodiments of this disclosure.

## DETAILED DESCRIPTION

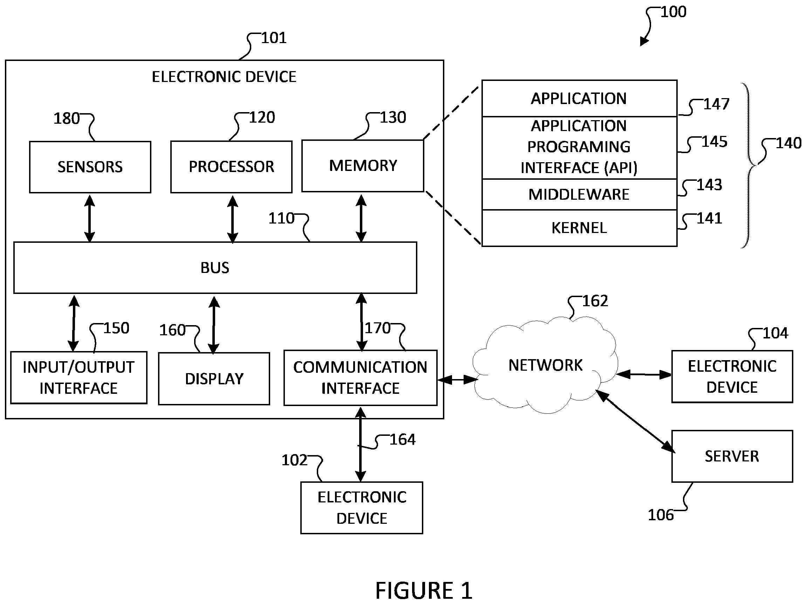

FIGURES 1 through 13, discussed below, and the various embodiments used to describe the principles of this disclosure in this patent document are by way of illustration only and should not be construed in any way to limit the scope of the disclosure. Those skilled in the art will understand that the principles of this disclosure can be implemented in any suitably arranged system.

FIGURE 1 illustrates an example network configuration 100 in accordance with this disclosure. As shown in FIGURE 1, according to embodiments of this disclosure, an electronic device 101 is included in the network configuration 100. The electronic device 101 may include at least one of a bus 110, a processor 120, a memory 130, an input/output (I/O) interface 150, a display 160, a communication interface 170, or a sensor 180. In some embodiments, the electronic device 101 may exclude at least one of the components or may add another component.

The bus 110 may include a circuit for connecting the components 120-180 with one another and transferring communications (such as control messages and/or data) between the components. The processor 120 may include one or more of a central processing unit (CPU), an application processor (AP), or a communication processor (CP). The processor 120 may perform control on at least one of the other components of the electronic device 101 and/or perform an operation or data processing relating to communication.

The memory 130 may include a volatile and/or non-volatile memory. For example, the memory 130 may store commands or data related to at least one other component of the electronic device 101. According to embodiments of this disclosure, the memory 130 may store software and/or a program 140. The program 140 may include, for example, a kernel 141, middleware 143, an application programming interface (API) 145, and/or an application program (or "application") 147. At least a portion of the kernel 141, middleware 143, or API 145 may be denoted an operating system (OS).

The kernel 141 may control or manage system resources (such as the bus 110, processor 120, or memory 130) used to perform operations or functions implemented in other programs (such as the middleware 143, API 145, or application program 147). The kernel 141 may provide an interface that allows the middleware 143, API 145, or application 147 to access the individual components of the electronic device 101 to control or manage the system resources. The middleware 143 may function as a relay to allow the API 145 or the application 147 to communicate data with the kernel 141, for example. A plurality of applications 147 may be provided. The middleware 143 may control work requests received from the applications 147, such as by allocating the priority of using the system resources of the electronic device 101 (such as the bus 110, processor 120, or memory 130) to at least one of the plurality of applications 147. The API 145 is an interface allowing the application 147 to control functions provided from the kernel 141 or the middleware 143. For example, the API 133 may include at least one interface or function (such as a command) for file control, window control, image processing, or text control.

The input/output interface 150 may serve as an interface that may, for example, transfer commands or data input from a user or other external devices to other component(s) of the electronic device 101. Further, the input/output interface 150 may output commands or data received from other component(s) of the electronic device 101 to the user or the other external devices.

The display 160 may include, for example, a liquid crystal display (LCD), a light emitting diode (LED) display, an organic light emitting diode (OLED) display, an active matrix OLED (AMOLED), a microelectromechanical systems (MEMS) display, or an electronic paper display. The display 160 can also be a depth-aware display, such as a multi-focal display. The display 160 may display various contents (such as text, images, videos, icons, or symbols) to the user. The display 160 may include a touchscreen and may receive, for example, a touch, gesture, proximity, or hovering input using an electronic pen or a body portion of the user.

The communication interface 170 may set up communication between the electronic device 101 and an external electronic device (such as a first electronic device 102, a second electronic device 104, or a server 106). For example, the communication interface 170 may be connected with a network 162 or 164 through wireless or wired communication to communicate with the external electronic device.

The electronic device 101 further includes one or more sensors 180 that can meter a physical quantity or detect an activation state of the electronic device 101 and convert metered or detected information into an electrical signal. For example, one or more sensors 180 can include one or more buttons for touch input, one or more cameras, a gesture sensor, a gyroscope or gyro sensor, an air pressure sensor, a magnetic sensor or magnetometer, an acceleration sensor or accelerometer, a grip sensor, a proximity sensor, a color sensor (such as a red green blue (RGB) sensor), a biophysical sensor, a temperature sensor, a humidity sensor, an illumination sensor, an ultraviolet (UV) sensor, an electromyography (EMG) sensor, an electroencephalogram (EEG) sensor, an electrocardiogram (ECG) sensor, an infrared (IR) sensor, an ultrasound sensor, an iris sensor, or a fingerprint sensor. The sensor(s) 180 can also include an inertial measurement unit, which can include one or more accelerometers, gyroscopes, and other components. The sensor(s) 180 can further include a control circuit for controlling at least one of the sensors included here. Any of these sensor(s) 180 can be located within the electronic device 101.

The first external electronic device 102 or the second external electronic device 104 may be a wearable device or an electronic device 101-mountable wearable device (such as a head mounted display (HMD)). When the electronic device 101 is mounted in an HMD (such as the electronic device 102), the electronic device 101 may detect the mounting in the HMD and operate in a virtual reality mode. When the electronic device 101 is mounted in the electronic device 102 (such as the HMD), the electronic device 101 may communicate with the electronic device 102 through the communication interface 170. The electronic device 101 may be directly connected with the electronic device 102 to communicate with the electronic device 102 without involving with a separate network.

The wireless communication may use at least one of, for example, long term evolution (LTE), long term evolution-advanced (LTE-A), code division multiple access (CDMA), wideband code division multiple access (WCDMA), universal mobile telecommunication system (UMTS), wireless broadband (WiBro), or global system for mobile communication (GSM), as a cellular communication protocol. The wired connection may include at least one of, for example, universal serial bus (USB), high definition multimedia interface (HDMI), recommended standard 232 (RS-232), or plain old telephone service (POTS). The network 162 may include at least one communication network, such as a computer network (like a local area network (LAN) or wide area network (WAN)), the Internet, or a telephone network.

The first and second external electronic devices 102 and 104 each may be a device of the same type or a different type from the electronic device 101. According to embodiments of this disclosure, the server 106 may include a group of one or more servers. Also, according to embodiments of this disclosure, all or some of the operations executed on the electronic device 101 may be executed on another or multiple other electronic devices (such as the electronic devices 102 and 104 or server 106). Further, according to embodiments of this disclosure, when the electronic device 101 should perform some function or service automatically or at a request, the electronic device 101, instead of executing the function or service on its own or additionally, may request another device (such as electronic devices 102 and 104 or server 106) to perform at least some functions associated therewith. The other electronic device (such as electronic devices 102 and 104 or server 106) may execute the requested functions or additional functions and transfer a result of the execution to the electronic device 101. The electronic device 101 may provide a requested function or service by processing the received result as it is or additionally. To that end, a cloud computing, distributed computing, or client-server computing technique may be used, for example.

While FIGURE 1 shows that the electronic device 101 includes the communication interface 170 to communicate with the external electronic device 102 or 104 or server 106 via the network(s) 162 and 164, the electronic device 101 may be independently operated without a separate communication function, according to embodiments of this disclosure. Also, note that the electronic device 102 or 104 or the server 106 could be implemented using a bus, a processor, a memory, an I/O interface, a display, a communication interface, and an event processing module (or any suitable subset thereof) in the same or similar manner as shown for the electronic device 101.

Although FIGURE 1 illustrates one example of a network configuration 100, various changes may be made to FIGURE 1. For example, the network configuration 100 could include any number of each component in any suitable arrangement. In general, computing and communication systems come in a wide variety of configurations, and FIGURE 1 does not limit the scope of this disclosure to any particular configuration. Also, while FIGURE 1 illustrates one operational environment in which various features disclosed in this patent document can be used, these features could be used in any other suitable system.

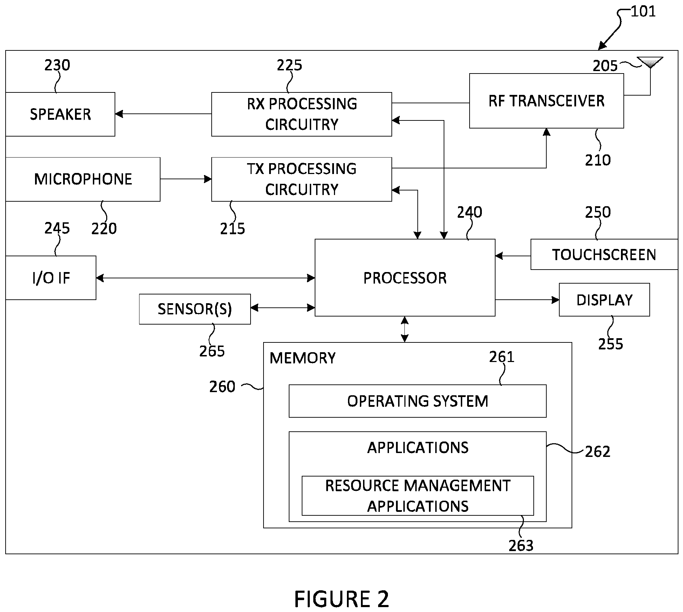

FIGURE 2 illustrates an example electronic device 101 according to embodiments of this disclosure. The electronic device 101 could represent one or more of the electronic devices 101, 102, or 104 in FIGURE 1. As shown in FIGURE 2, the electronic device 101 includes an antenna 205, a radio frequency (RF) transceiver 210, transmit (TX) processing circuitry 215, a microphone 220, and receive (RX) processing circuitry 225. The electronic device 101 also includes a speaker 230, a processor 240, an input/output (I/O) interface (IF) 245, an input 250, a display 255, and a memory 260. The memory 260 includes an operating system (OS) program 261 and one or more applications 262.

The RF transceiver 210 receives, from the antenna 205, an incoming RF signal transmitted by another component in a system. The RF transceiver 210 down-converts the incoming RF signal to generate an intermediate frequency (IF) or baseband signal. The IF or baseband signal is sent to the RX processing circuitry 225, which generates a processed baseband signal by filtering, decoding, and/or digitizing the baseband or IF signal. The RX processing circuitry 225 transmits the processed baseband signal to the speaker 230 (such as for voice data) or to the processor 240 for further processing (such as for web browsing data).

The TX processing circuitry 215 receives analog or digital voice data from the microphone 220 or other outgoing baseband data (such as web data, e-mail, or interactive video game data) from the processor 240. The TX processing circuitry 215 encodes, multiplexes, and/or digitizes the outgoing baseband data to generate a processed baseband or IF signal. The RF transceiver 210 receives the outgoing processed baseband or IF signal from the TX processing circuitry 215 and up-converts the baseband or IF signal to an RF signal that is transmitted via the antenna 205.

The processor 240 can include one or more processors or other processors and execute the OS program 261 stored in the memory 260 in order to control the overall operation of the electronic device 101. For example, the processor 240 could control the reception of forward channel signals and the transmission of reverse channel signals by the RF transceiver 210, the RX processing circuitry 225, and the TX processing circuitry 215 in accordance with well-known principles. In some embodiments, the processor 240 includes at least one microprocessor or microcontroller.

The processor 240 is also capable of executing other processes and programs resident in the memory 260. The processor 240 can move data into or out of the memory 260 as required by an executing process. In some embodiments, the processor 240 is configured to execute the applications 262 based on the OS program 261 or in response to signals received from external devices or an operator. The processor can execute a resource management application 263 for monitoring system resources. The processor 240 is also coupled to the I/O interface 245, which provides the electronic device 101 with the ability to connect to other devices such as laptop computers, handheld computers and other accessories, for example, a VR headset. The I/O interface 245 is the communication path between these accessories and the processor 240. The processor 240 can recognize accessories that are attached through the I/O interface 245, such as a VR headset connected to a USB port.

The processor 240 is also coupled to the input 250 and the display 255. The operator of the electronic device 101 can use the input 250 (e.g., keypad, touchscreen, button etc.) to enter data into the electronic device 101. The display 255 may be an LCD, LED, OLED, AMOLED, MEMS, electronic paper, or other display capable of rendering text and/or at least limited graphics, such as from web sites.

The memory 260 is coupled to the processor 240. Part of the memory 260 could include a random access memory (RAM), and another part of the memory 260 could include a Flash memory or other read-only memory (ROM).

The electronic device 101 further includes one or more sensors 265 that can meter a physical quantity or detect an activation state of the electronic device 101 and convert metered or detected information into an electrical signal. For example, the sensor 265 may include any of the various sensors 180 discussed above.

Although FIGURE 2 illustrates one example of an electronic device 101, various changes may be made to FIGURE 2. For example, various components in FIGURE 2 could be combined, further subdivided, or omitted and additional components could be added according to particular needs. As a particular example, the processor 240 could be divided into multiple processors, such as one or more central processing units (CPUs) and one or more graphics processing units (GPUs). Also, while FIGURE 2 illustrates the electronic device 101 configured as a mobile telephone or smartphone, electronic devices could be configured to operate as other types of mobile or stationary devices. In addition, as with computing and communication networks, electronic devices can come in a wide variety of configurations and FIGURE 2 does not limit this disclosure to any particular electronic device.

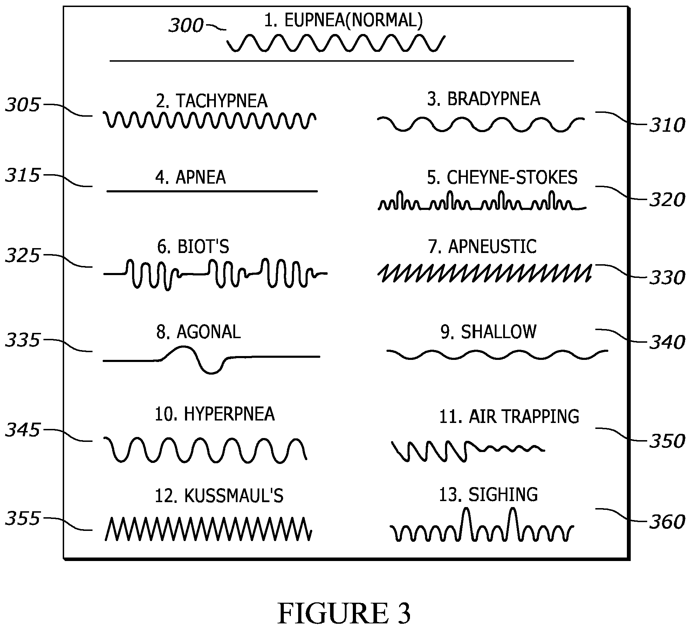

FIGURE 3 illustrates examples of breathing waveforms for different types of pulmonary conditions according to embodiments of this disclosure. Various pulmonary conditions present distinct and unique waveforms that can aid in identifying a pulmonary condition that a user is experiencing. As illustrated in FIGURE 3, breathing patterns are provided for Tachypnea 305, Bradypnea 310, Apnea 315, Cheyne-Stokes 320, Biot's 325, Apneustic 330, Agonal 335, Shallow 340, Hypernea 345, air trapping 350, Kussmaul's 355, and sighing 360. These patterns 305-360 present differently when compared to the breathing pattern of Eupnea 300, which is a normal breathing pattern. However, several of the breathing patterns 305-360 can also be found with healthy users during exercise or in conversation. Therefore, a user's condition may not always be reliably estimated based on the breathing waveform alone.

According to the claimed invention, audio data is received by a separate microphone. The microphone 220 can be placed on various parts of a user's body, such as the user's chest, abdomen, mouth, or back of the sternum.

An identification of the respiratory phases of a user's breathing pattern can be useful when detecting adventitious sounds to identify a pulmonary condition, including a severity of the condition, of a user. For example, wheezing during an inspiration phase may indicate a higher level of severity compared to when wheezing happens in an expiration phase. Moreover, severity of patient condition worsens when wheezing happens in both phases. Embodiments of the invention therefore identify respiratory phases by applying appropriate signal processing steps on audio data.

Although FIGURE 3 illustrates examples of breathing waveforms for different types of pulmonary conditions, various changes may be made to FIGURE 3. For example, the specific waveforms shown in FIGURE 3 are for illustration only.

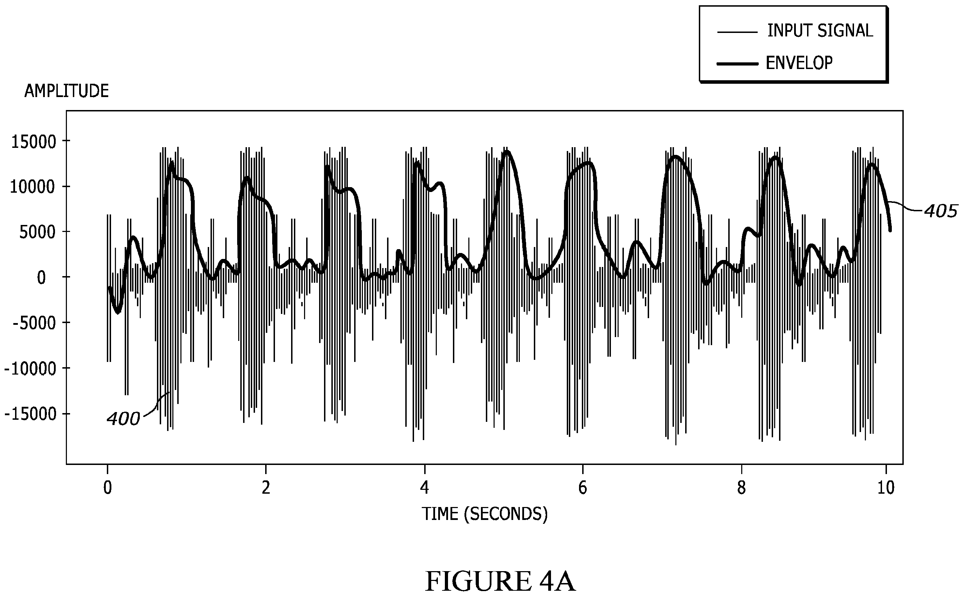

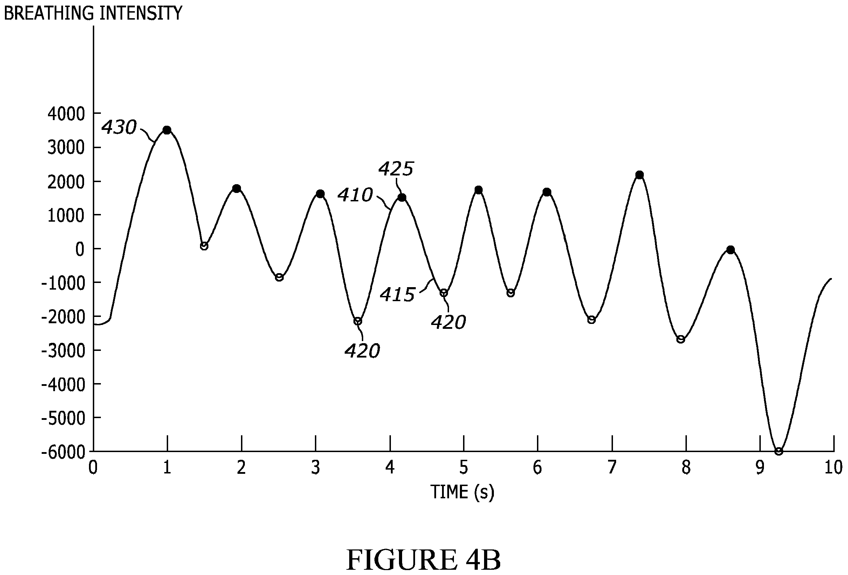

FIGURES 4A and 4B illustrate an example respiratory phase analysis of audio breathing data according to embodiments of this disclosure. In particular, FIGURE 4A illustrates one-dimensional audio breathing data 400 in the time-domain and an envelope signal 405 produced by an algorithm according to embodiments of this disclosure. Respiratory phases (that is, inspiration and expiration) can be identified by the envelope signal 405 of the audio breathing data 400 in the time-domain when they are prominent in the audio data 400. Thus, according to some embodiments, an envelope detection algorithm is applied to automatically detect the start and end of each respiratory phase. In particular embodiments, the algorithm can operate as follows. The algorithm detects peaks in the audio data 400 and applies a cubic spline curve fitting model to the peaks. Since audio sampling frequency and variation of the audio data 400 in the time-domain are usually very high, the algorithm applies the same envelop detection algorithm on the peaks detected in the prior step. A third-order low-pass Butterworth filter with a cut-off frequency of 1.66 HZ is applied to further smooth the envelop signal 405.

FIGURE 4B illustrates a resulting audio data signal 430 after the envelope detection algorithm is applied. Based on the resulting signal 430, an inspiration phase 410 is determined between the timestamp of a trough 420 (start of inspiration) and the timestamp of a following peak 425 (end of inspiration). Similarly, an expiration phase 415 is determined as the timestamp of a peak 425 (start of expiration) and the timestamp of a following trough 420 (end of expiration). The resulting audio data signal 430 can be dynamically segmented into variable lengths and converted from a time-domain signal into a spectro-temporal image using the start-time and end-time of the respiratory phases.

Although FIGURES 4A and 4B illustrate an example of a respiratory phase analysis of audio breathing data, various changes may be made to FIGURES 4A and 4B. For example, the signals and data shown in FIGURES 4A and 4B are for illustration only and merely meant to illustrate how audio breathing data can be analyzed.

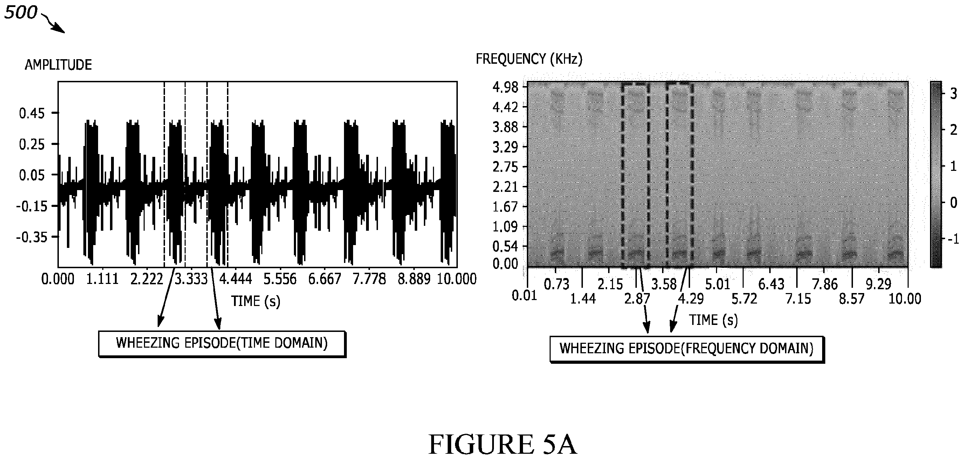

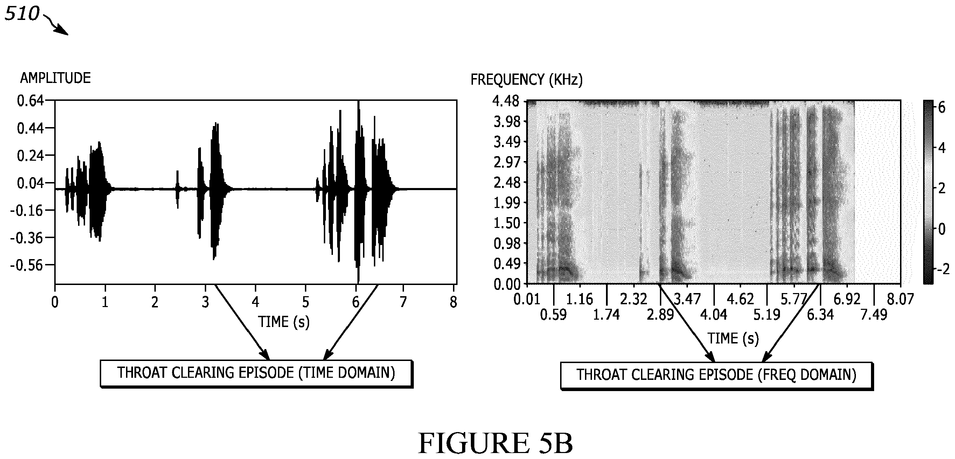

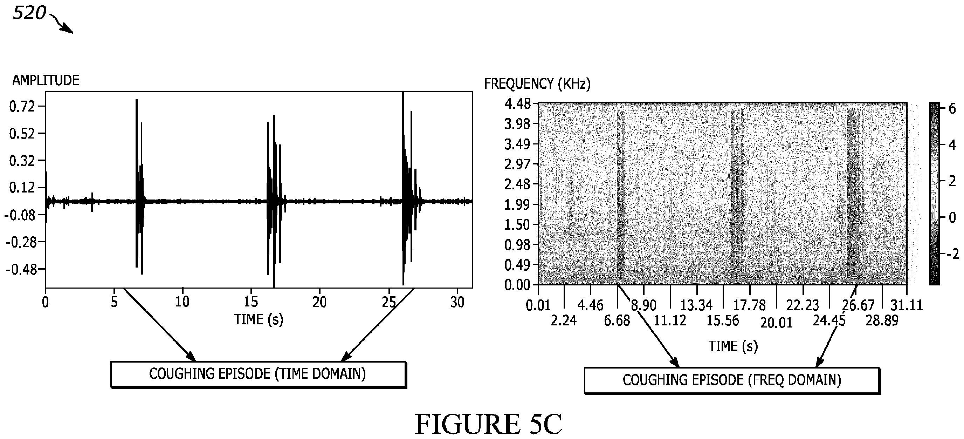

FIGURES 5A, 5B, and 5C illustrate example types of audio breathing data in a time domain and in a frequency domain (such as in a spectro-temporal image) according to embodiments of this disclosure. As previously discussed, different user conditions can present using different breathing waveforms. Similarly, different user conditions can present based on different audio data or signals. As shown in FIGURES 5A, 5B, and 5C, audio data of a wheezing episode 500 presents very differently from audio data of a throat clearing episode 510 or of a coughing episode 520. Therefore, according to embodiments of this disclosure, audio data (such as the audio data 400) can be converted into spectro-temporal image data, which can then be processed through a convolutional neural network (CNN)-based deep learning model or other machine learning algorithms to classify sounds. Other embodiments can utilize both time domain data and converted spectro-temporal data to classify abnormal body sounds regarding chronic patients, such as asthma or COPD patients.

Although FIGURES 5A, 5B, and 5C illustrate example types of audio breathing data in a time domain and in a frequency domain, various changes may be made to FIGURES 5A, 5B, and 5C. For example, the waveforms, images, and other data shown in FIGURES 5A, 5B, and 5C are for illustration only.

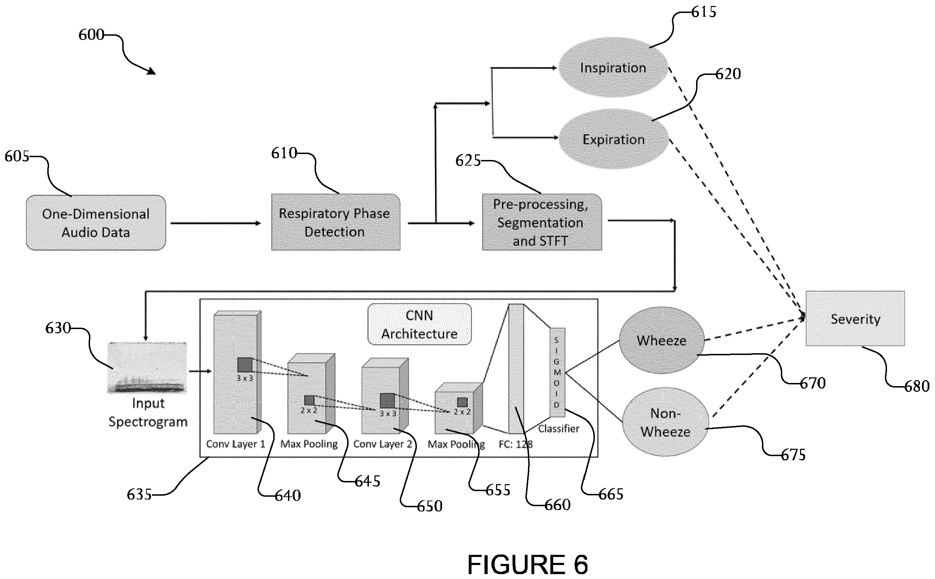

FIGURE 6 illustrates an example architecture 600 for wheeze detection using audio breathing data according to embodiments of this disclosure. One-dimensional audio data 605 here is provided to a respiratory phase detection function 610, which operates to identify inspiration phases 615 and expiration phases 620 in a user's breathing. The respiratory phase detection function 610 can use any suitable technique to identify the inspiration phases 615 and expiration phases 620, such as the technique described above.

The audio data 605 and the respiratory phases are provided to a pre-processing, segmentation, and transformation function 625. For example, the function 625 can filter the audio data 605, segment the audio data 605 based on the identified respiratory phases, and transform the audio data 605 into at least one spectro-temporal image or spectrogram 630. The segmentation of the audio data 605 can divide the audio data 605 into different segments for different inspiration and expiration phases. The audio data 605 can undergo any suitable transformation to generate the spectrogram 630, such as a Short Term Fourier Transformation (STFT). In particular embodiments, the audio data 605 can be segmented based on a variable time window of the user's respiratory phase. In other embodiments, the audio data 605 can be segmented into different window sizes such as 0.25s, 0.5s, 0.75s, 0.85s, 1s, etc. to find the best-performing window size. Other embodiments can use natural, dynamic windows based on the start and end of the respiratory phases or respiratory cycles to capture atomic abnormal pulmonary sounds (such as wheeze, crackle, ronchi, cough).

Spectrograms from different sources of audio data may have completely different characteristics. For example, the sampling rate may be different across multiple sources of audio data, which produce spectrograms of different spectral resolutions. According to embodiments of this disclosure, an algorithm computes a low-pass filter with a cut-off frequency of 4 kHz or other value above the dominant frequency of wheezing (which is between 400 Hz to 2.5 kHz). The sampling rate of the audio data is then normalized. For example, if the sampling rate of the audio data is more than 9 kHz, the audio data can be down-sampled to 9 kHz to ensure uniformity of the audio signal characteristics. The algorithm also applies a high-pass filter, such as with a cut-off frequency of 200 Hz, on the down-sampled signal (since adventitious sounds such as rhonchus and crackle have a frequency below 300 Hz).

Note that the spectrum variation of a non-stationary signal that may not be captured in a single Fourier analysis can be generated by computing the Fourier transform of shorter slices (considered to be stationary) of the segmented signal. Thus, in some embodiments, during the spectrogram computation a sliding Hann window of length W=256 can be applied to 50% overlapping segments of the signal given by the following.

$$(1) \qquad x[n,k] = \sum_{m=0}^{w-1} x[m]w[n-m]e^{-i\frac{2\pi k}{N}m}$$

The spectrogram 630 can then be computed as the magnitude squared of the STFT, which is given by the following.

$$(2) \qquad \text{Spectrogram } \{x(t)\}(n,k) = |X[n,k]|^2$$

As shown in FIGURE 6, the spectrogram 630 is passed through a CNN-based deep learning model 635 to classify the sounds represented by the spectrogram 630. In this example, the deep learning model 635 contains two convolution layers 640 and 650, two max pooling layers 645 and 655, one fully-connected layer 660, and a classification layer 665. The output of the max-pooling layer 655 is processed by the fully-connected layer 660. Finally, the classification layer 665 (such as a sigmoid layer) classifies sounds, such as by identifying (for each sound) a "wheeze" indicator 670 or a "non-wheeze" indicator 675. This embodiment can therefore identify a pulmonary condition of a user as asthma based on the detection of a "wheeze." Other embodiments may use different learning parameters to create optimal algorithms for training models to classify other sounds. For example, other adventitious sounds to be detected may include wheeze or ronchi, stridor, inspiratory gasp, crackles among other distinguishing sounds. In some embodiments, the classification layer 665 can be implemented using sigmoid neurons.

Some embodiments of this disclosure may determine the pulmonary condition of a user based on the respiratory phase 615, 620 in which an adventitious sound occurs, in addition to the type of adventitious sound detected by the deep learning model(s) 635. Other embodiments of this disclosure may determine a severity 680 of the detected pulmonary condition. The severity 680 can be based on various factors, such as the respiratory phase(s) 615, 620, the adventitious sound(s) detected, and other distinguishing features like inspiration to expiration ratio, a duration of the abnormal sound, an intensity of the abnormal sound, and/or a user's baseline condition (as explained below).

The various functions and operations shown and described above with respect to FIGURE 6 can be implemented in at least one electronic device (such as any of the electronic devices 101, 102, 104 or the server 106) in any suitable manner. For example, in some embodiments, at least some of the functions and operations can be implemented or supported using one or more software applications or other software instructions that are executed by the processor(s) 120, 240 of the electronic device(s). In other embodiments, at least some of the functions and operations can be implemented or supported using dedicated hardware components. In general, the functions and operations can be performed using any suitable hardware or any suitable combination of hardware and software/firmware instructions.

Although FIGURE 6 illustrates one example of an architecture 600 for wheeze detection using audio breathing data, various changes may be made to FIGURE 6. For example, the deep learning model 635 can be implemented using any other suitable deep learning-based machine learning algorithm, such as a Long Short Term Memory (LSTM) or other machine learning algorithm or combination thereof. Also, the architecture 600 shown in FIGURE 6 could be implemented in any suitable manner, such as entirely within an electronic device 101 or using a combination of devices. For instance, an electronic device 101 could collect the audio data 605 and provide the audio data 605 to a server 106, which could then process the data as shown in FIGURE 6. Results of the processing could then be made available to the electronic device 101 and/or one or more other devices (such as the electronic device 102 or 104).

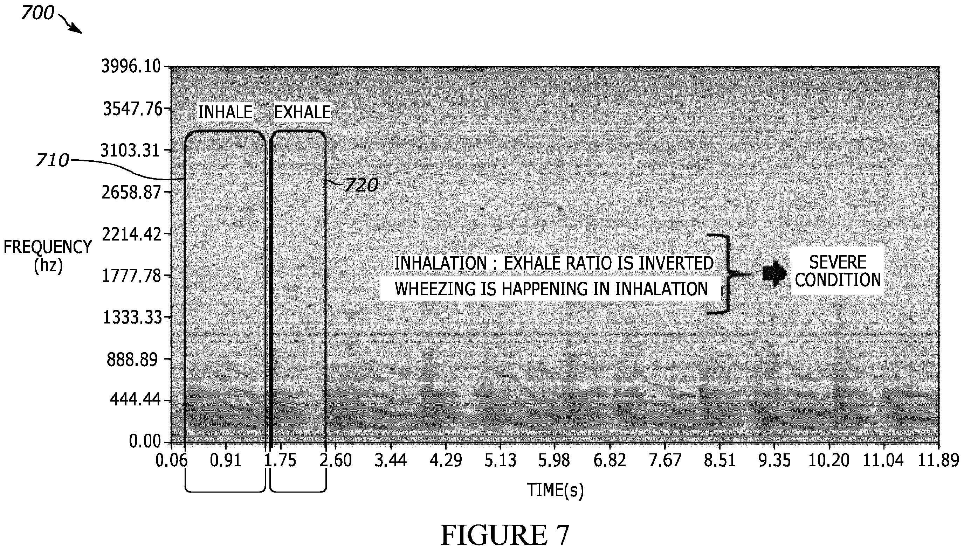

FIGURE 7 illustrates an example spectrogram 700 of a severe wheezing episode as detected in a frequency domain chart according to embodiments of this disclosure. In particular, the spectrogram 700 of the wheezing episode has an inspiration phase 710 and an expiration phase 720 whose durations are inverted. The inspiration phase 710 and the expiration phase 720 of audio data can be identified as discussed above, and the time period of each phase can be identified in the spectrogram 700. This example shows that wheezing is happening in both the inspiration and expiration phases and that the inspiration-to-expiration (IE) ratio is inverted. That is, under normal conditions, the expiration phase 720 is longer in time than the inspiration phase 710. Accordingly, some embodiments can use additional factors such as the IE ratio being inverted and wheezing occurring in both phases to determine a severity of a specific pulmonary condition (asthma in this example). The duration and intensity (corresponding to the frequency) of the adventitious sound can also be identified from the spectrogram 700.

Although FIGURE 7 illustrates one example of a spectrogram 700 of a severe wheezing episode as detected in a frequency domain chart, various changes may be made to FIGURE 7. For example, other pulmonary conditions (besides asthma) can have other characteristics that are detectable using a spectrogram or other information.

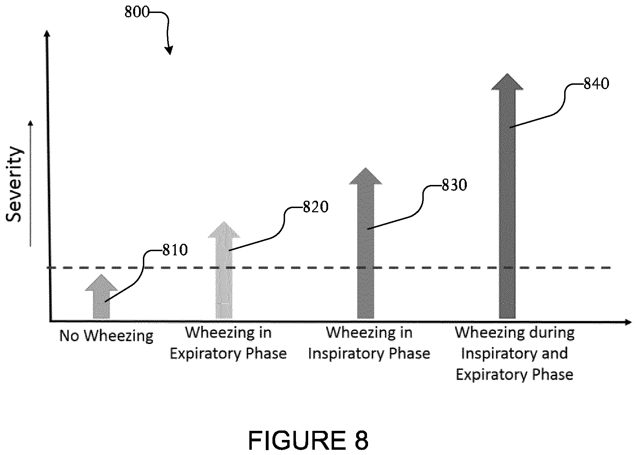

FIGURE 8 illustrates an example severity chart 800 for a pulmonary patient according to embodiments of this disclosure. In this example, no wheezing 810 would indicate that the user is not currently experiencing pulmonary symptoms. Wheezing in the expiration phase 820 may indicate that the user is beginning to experience pulmonary symptoms or that the symptoms are mild. Wheezing in the inspiratory phase 830 may indicate that the user's symptoms are somewhat severe and may be a cause for concern. Wheezing in the inspiratory and expiratory phases 840 may indicate that the pulmonary patient's symptoms are severe and that an alert is required. It is therefore possible for a system to be configured to analyze audio data in the manner described above (generating and analyzing a spectrogram to identify adventitious sounds and the respiratory phases in which the adventitious sounds are occurring) and identify both the pulmonary condition affecting the user and the severity of the pulmonary condition.

This is just one example of how embodiments of this disclosure can gauge the severity of a pulmonary condition. Other factors can also be monitored to determine the severity of various pulmonary conditions. Outside factors, such as environment, user activity, and so forth, are other ways that the severity of a pulmonary condition can be monitored. Various embodiments may rate the severity of various pulmonary conditions based on a variety of factors and symptoms as disclosed here.

Although FIGURE 8 illustrates one example of a severity chart 800 for a pulmonary patient, various changes may be made to FIGURE 8. For example, there may be other factors that can be used to identify other or additional severities of a pulmonary condition.

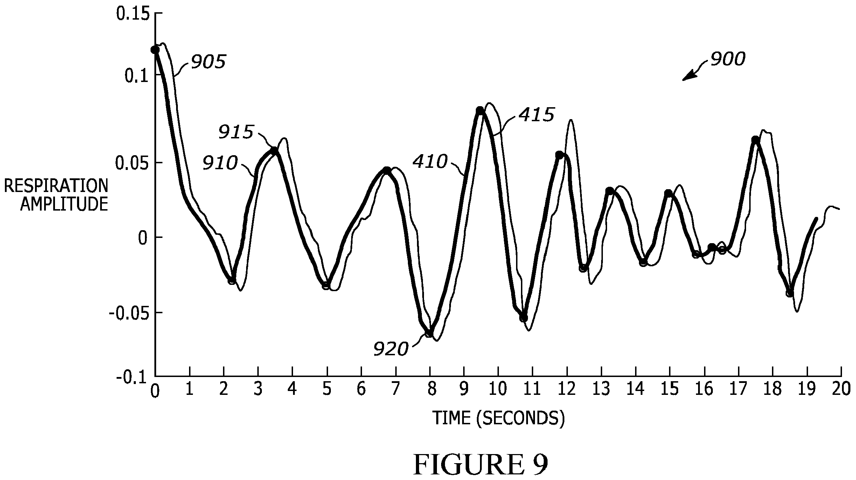

FIGURE 9 illustrates example breathing waveform data and respiratory phase information 900 from at least one motion sensor according to embodiments of this disclosure. In some situations, the audio data captured by a microphone 220 of an electronic device 101 may be adequate to identify the respiratory phases of a user's breathing. In other situations (such as when large amounts of background noise affect the quality of the audio data), it may be necessary or desirable to supplement the audio data with motion sensor data or other data. The motion sensor data could be collected by one or more sensors 180, 265 of the electronic device 101. The motion sensor data could also or alternatively be collected by one or more other electronic devices that are placed on one or more parts of the user's body, such as the chest or abdomen. Example types of motion sensors that could be used here may include 3D accelerometers, gyroscopes, or other inertial motion sensors.

As shown in FIGURE 9, raw motion sensor data 905 may go through signal processing steps similar to what was discussed above for the audio data. The signal processing steps can smooth the raw motion sensor data 905 to produce a breathing waveform 910. Peaks 915 and troughs 920, as well as inspiration phases 410 and expiration phases 415, of the breathing waveform 910 can then be identified.

According to the invention, the breathing waveform data is used to identify a pulmonary condition of the user. According to the invention, the breathing waveform 910 and the identified respiratory phases 410, 415 are used in conjunction with the abnormal sound assocaited with the user's breathing based on the image data which was generated from the audio data to identify a pulmonary condition of a user. Variations of the breathing patterns shown in FIGURE 9 can be used to diagnose pulmonary and non-pulmonary patient conditions, such as asthma, COPD, or heart attack.

Several variations of the pathological, abnormal breathing patters are shown in FIGURE 3 and described above.

Although FIGURE 9 illustrates one example of a breathing waveform data and respiratory phase information 900 from at least one motion sensor, various changes may be made to FIGURE 9. For example, the waveforms shown in FIGURE 9 are for illustration only.

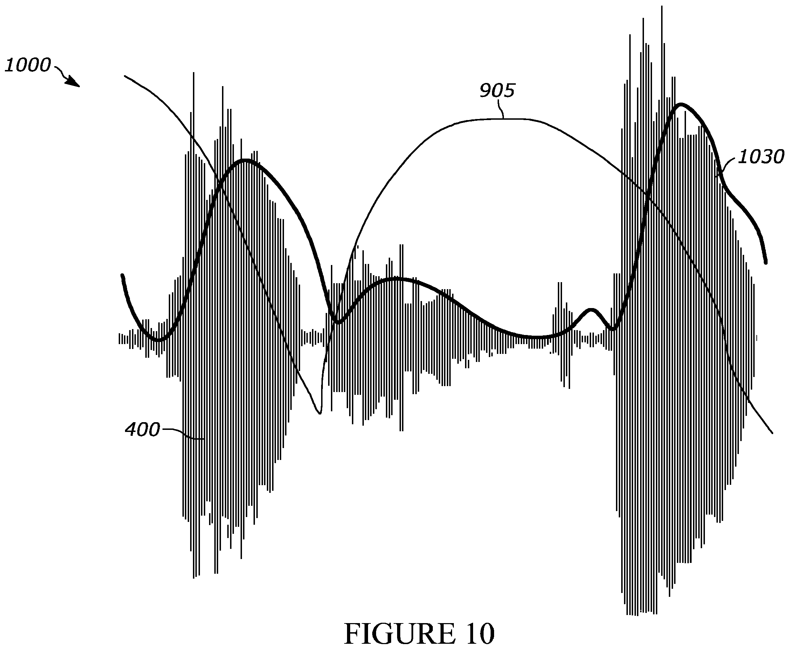

FIGURE 10 illustrates example audio data 400 and motion data 905 collected from different sensors according to embodiments of this disclosure. For example, the audio data 400 may be captured from a microphone 220 of a user device 101 or another electronic device 102, 104. Also, the motion data 905 may be captured from at least one motion sensor 180, 265 of a user device 101 or other device(s) (which may be placed on different parts of a user's body). However, the various pieces of data may not always correspond with each other. Therefore, it may be necessary or desirable to synchronize the audio data 400 and the motion data 905 for accurate results. This can be particularly useful when the data comes from multiple sources.

In some embodiments, data from multiple sources is first analyzed to ensure that the data is coming from reliable data sources. As represented in FIGURE 10, for example, data collected from reliable data sources show a distinct pattern of inhalation and exhalation both in the audio data 400 and the motion data 905. Data from non-reliable sources can be excluded, and subsets of audio data 400 and/or motion data 905 (from the reliable sources) can be synchronized and processed. Thus, the patterns in the audio data 400 and the motion data 905 can be synchronized, such as by using a time-series matching algorithm like shapelet and sequence matching. A resulting sound envelope signal 1030 can be used as the input signal to undergo signal processing, which outputs a breathing waveform used to identify respiratory phases 410, 415 as well as abnormal breathing patterns. Abnormal breathing patterns identified from the breathing waveform 910 are also used to identify a pulmonary condition of the user.

Although FIGURE 10 illustrates one example of audio data 400 and motion data 905 collected from different sensors, various changes may be made to FIGURE 10. For example, the data shown in FIGURE 10 is for illustration only. Also, more than two signals can be synchronized and used to identify a pulmonary condition or its severity.

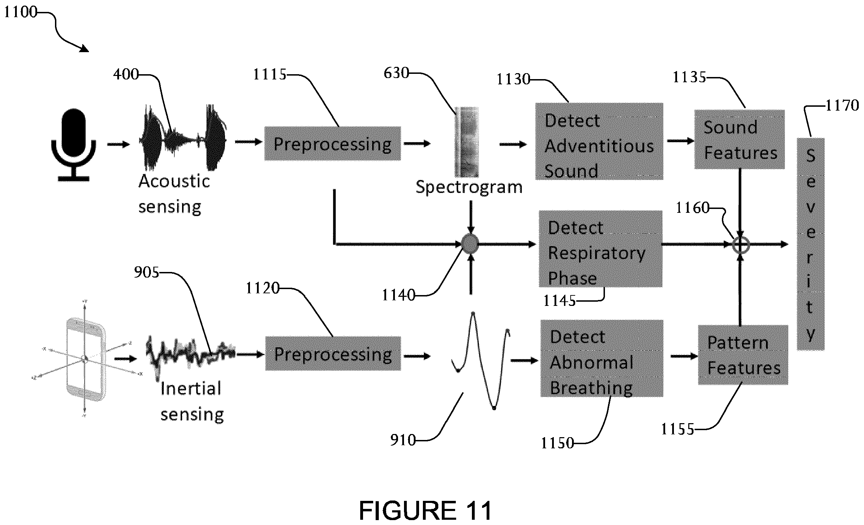

FIGURE 11 illustrates an example architecture 1100 for determining a pulmonary condition of a user and a severity of the pulmonary condition based on audio data and motion data according to embodiments of this disclosure. As shown in FIGURE 11, audio data 400 and motion data 905 of a user are gathered. As noted above, the audio data 400 can be collected using the microphone 220 of an electronic device 101, and the motion data 905 can be collected using the sensor(s) 180, 265 of the electronic device 101 or using other sensors. A pre-processing operation 1115 applies one or more pre-processing algorithms to the audio data 400, as previously described, to create at least one spectrogram 630. A pre-processing operation 1120 applies one or more pre-processing algorithms to the motion data 905, as previously described, to create at least one breathing waveform 910.

A detection function 1130 analyzes the spectrogram 630 to identify one or more sound features 1135 of the audio data. The detection function 1130 could, for example, implement the CNN-based deep learning model 635 or other machine learning model to analyze the spectrogram 630. Any adventitious sound identified by the detection function 1130 can be identified in the resulting sound features 1135. Other features of the adventitious sounds, such as their intensity and duration, can also be identified in the sound features 1135.

A synchronization function 1140 synchronizes audio-related data and waveform-related data, such as by using the approach described above based on the inspiration and expiration phases contained in the data. The synchronized data is provided to a detection function 1145, which analyzes the synchronized data to identify the respiratory phases (inspiration and expiration phases).

A detection function 1150 processes the breathing waveform 910 to identify pattern features 1155 of the user's breathing. The detection function 1150 could, for example, determine an inspiration-to-expiration ratio of the breathing waveform 910 or any other abnormalities associated with the breathing waveform 910. Any abnormalities associated with the breathing waveform 910 can be identified in the resulting pattern features 1155.

The sound features 1135, detected respiratory phases, and pattern features 1155 are provided to an analysis function 1160, which generates an output 1170 identifying a pulmonary condition of the user and a severity of the pulmonary condition. As noted above, this can take various forms. With respect to wheezing, for example, the analysis function 1160 can determine whether wheezing is occurring, in which respiratory phase(s) the wheezing is occurring, and whether the user's inspiration and expiration phases are inverted (indicating a possibly severe pulmonary condition). Additional factors, such as environment and user input, may also be used as part of the analysis function 1160.

The various functions and operations shown and described above with respect to FIGURE 11 can be implemented in at least one electronic device (such as any of the electronic devices 101, 102, 104 or the server 106) in any suitable manner. For example, in some embodiments, at least some of the functions and operations can be implemented or supported using one or more software applications or other software instructions that are executed by the processor(s) 120, 240 of the electronic device(s). In other embodiments, at least some of the functions and operations can be implemented or supported using dedicated hardware components. In general, the functions and operations can be performed using any suitable hardware or any suitable combination of hardware and software/firmware instructions.

Although FIGURE 11 illustrates one example of an architecture 1100 for determining a pulmonary condition of a user and a severity of the pulmonary condition based on audio data and motion data, various changes may be made to FIGURE 11. For example, the architecture 1100 shown in FIGURE 11 could be implemented in any suitable manner, such as entirely within an electronic device 101 or using a combination of devices. For instance, an electronic device 101 could collect the data 400, 905 and provide the data 400, 905 to a server 106, which could then process the data as shown in FIGURE 11. Results of the processing could then be made available to the electronic device 101 and/or one or more other devices (such as the electronic device 102 or 104).

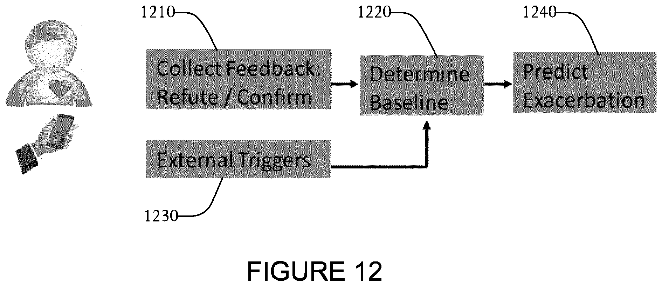

FIGURE 12 illustrates an example process 1200 of predicting a user's pulmonary condition and severity using baseline data according to embodiments of this disclosure. In some embodiments, a user's condition can be monitored repeatedly or continuously using the approaches described above. For example, the user's electronic device 101 may repeatedly capture audio data (and optionally motion data) over time. This data can be stored for a period on the user's electronic device 101 or streamed from the electronic device 101 to another device (such as the server 106). This data can be analyzed by the electronic device 101, server 106, or other device to establish a baseline condition for the user.

As shown in FIGURE 12, a collection function 1210 can be used to collect data about the user and to collect feedback from the user. The feedback can be used to indicate whether the collected user data is indicative of a breathing problem (as determined by the user). As particular examples, the feedback could be used to identify whether collected data is associated with an episode of a breathing problem, exercise, or conversation. A determination function 1220 analyzes the collected data to identify the user's baseline condition. For example, the determination function 1220 can analyze the collected data to determine normal characteristics of the user's breathing (such as in terms of audio data characteristics and/or motion data characteristics). The user's electronic device 101 may also query the user for feedback regarding whether or not a detected severity level is true or false for the user. In this way, the baseline condition may vary from user to user and can additionally change for the same user over time.

Various other features, such as external triggers 1230, can be collected and provided to the determination function 1220 and incorporated into the user's baseline condition. For example, a user can input environmental changes, such as allergens or weather, that may affect a pulmonary condition. The user can also provide input regarding what type of activity a user is participating in. At least some of this information may also be collected automatically, such as when weather or allergen data is collected from weather monitoring stations or other sources or physical activity data is collected from the mobile devices carried by the user (such as a smartphone or wrist-worn devices). Background noise and other variables can also be considered.

Once a baseline condition is established for a user, a prediction function 1240 can be used to collect additional information about the user (such as additional audio and/or motion data) and predict if the user is likely to experience an exacerbation or attack. If so, an alert or other notification can be sent to the user's electronic device 101 or to other destination(s) (such as one or more other electronic devices 102 and 104).

In some embodiments, a "risk score" can be repeatedly generated for a user over time based on the information collected about the user. The risk scores can then be used to predict the likelihood of time to an exacerbation or attack, and (if necessary) an alert or other notification when the risk score is above a specified threshold. For example, the following equation could be used when determining the risk score.

$$(3) \qquad h(t|x) = h_0(t)e^{((\beta\_1 * x\_1) + ... + (\beta\_p * x\_p))}$$

Equation (3) here models the risk at time $t$ given a set of predictors $x_1$, $x_2$, ..., $x_p$ (such as blood oxygen saturation, respiratory rate, heart rate, temperature, and air pollution). Each predictor has a corresponding coefficient $\beta_1$, $\beta_1$, ..., $\beta_p$, and each coefficient measures the impact of the corresponding covariate. The baseline condition of the patient is denoted $h_0(t)$, which corresponds to the state of the patient if all $x_i$ values are equal to 0. The instantaneous risk is given by $e^{((\beta\_1 * x\_1) + ... + (\beta\_p * x\_p))}$ given the set of predictors at time $t$. The risk varies over time, which is shown by the time variable $t$ in the expression $h(t|x)$.

This functionality may be combined with the functionality described above to both identify a current condition of a user and predict a future condition of the user. In other words, the various approaches described above with respect to FIGURES 1 through 11 can be used to collect and analyze data to identify the current condition of the user. The approach shown in FIGURE 12 can be used to collect and analyze data to predict the future condition of the user.

The various functions and operations shown and described above with respect to FIGURE 12 can be implemented in at least one electronic device (such as any of the electronic devices 101, 102, 104 or the server 106) in any suitable manner. For example, in some embodiments, at least some of the functions and operations can be implemented or supported using one or more software applications or other software instructions that are executed by the processor(s) 120, 240 of the electronic device(s). In other embodiments, at least some of the functions and operations can be implemented or supported using dedicated hardware components. In general, the functions and operations can be performed using any suitable hardware or any suitable combination of hardware and software/firmware instructions.

Although FIGURE 12 illustrates one example of a process 1200 of predicting a user's pulmonary condition and severity using baseline data, various changes may be made to FIGURE 12. For example, any of the operations shown in FIGURE 12 could be repeated any number of times.

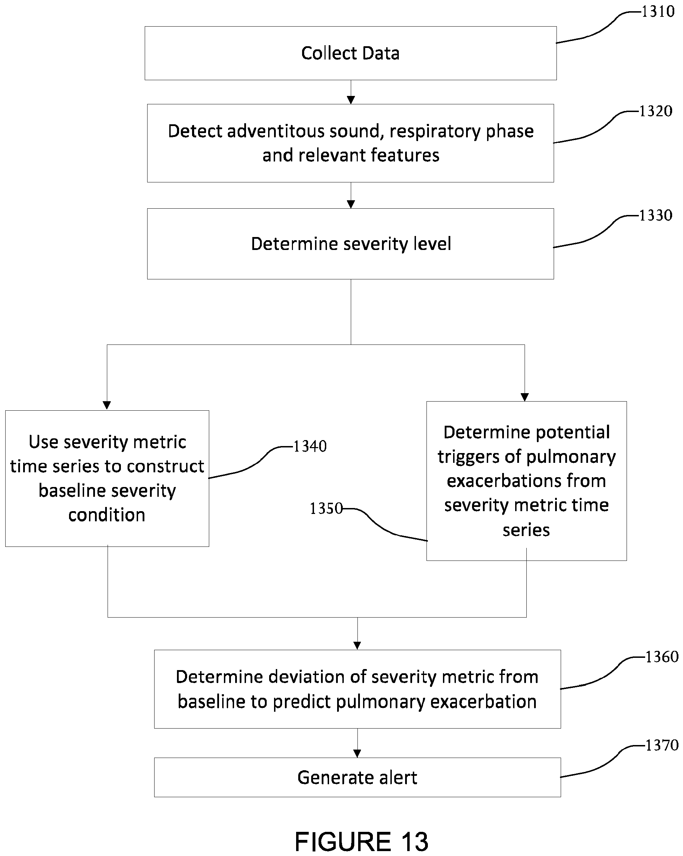

FIGURE 13 illustrates an example method 1300 of issuing an alert based on collection of pulmonary data according to embodiments of this disclosure. In step 1310, data associated with a user is collected over time. This data may include audio data and/or motion data collected from an electronic device 101 or from multiple sensors or other devices. In some cases, data collection may occur in an active mode in which the user actively measures his or her breathing activity using the electronic device 101 or other devices. For example, the user may place the electronic device 101 on the user's chest wall or keep the electronic device 101 in close proximity to the user's mouth or nose. Data collection may also occur in passive mode in which audio data (and possibly other data) is collected without the user's explicit participation, such as when the user's electronic device 101 collects audio data of the user's breathing while the user is not using the electronic device 101. Combinations of modes can also be used, where some data is collected actively while other data is collected passively.

In step 1320, adventitious sounds, respiratory phases, and other relevant features of the user's breathing are detected. For example, when used in the active mode, the user's respiration phases can be detected using an accelerometer or other sensor 180, 265 of the electronic device 101, and abnormal waveforms can be detected based on a breathing waveform 910 that is generated from motion data. As another example, the microphone 220 of the electronic device 101 can capture audio data, and abnormal sounds and respiratory phases may be detected from the audio data. Other relevant features may also be identified from the audio or motion data during this step.

When used in the passive mode, audio and motion data may be collected based on the user's interactions with the electronic device 101. For example, the microphone 220 of the electronic device 101 could capture audio data used to detect the respiratory phases of the user's breathing while the user is participating in a telephone call. Abnormal lung sounds during the user's inspiration and expiration phases may be distinguished from speech as described above. As another example, if the electronic device 101 is placed near a user, sounds such as typing or speech from another person can be distinguished from abnormal breathing sounds.

Note that in any operational mode, a combination of sensors can be used at the same time to collect audio data, motion data, or other data associated with the user that is then processed (possibly after being synchronized). The ability to collect data from multiple sources helps to increase the accuracy of the pulmonary condition detection. The placement and use of the various devices and sensors can vary from user to user.

In step 1330, a severity level of a pulmonary condition of the user is identified based on the adventitious sounds, respiratory phases, and relevant features identified previously. According to embodiments of this disclosure, the severity level can be determined differently for various types of pulmonary conditions.

In step 1340, a baseline severity condition for a user is established. That is, as data is collected over time, the user can input data identifying whether present conditions are causing the user to experience increased pulmonary condition severity. A severity metric time series may therefore be created from this collected data. Eventually, a baseline severity condition of the user is established, which is helpful since various factors affect different users in different ways. In step 1350, the data collected over time is analyzed to determine whether there may be outside triggers, such as environmental changes, affecting the pulmonary condition of the user. This could include, for instance, collecting data from weather stations or other sources to determine whether certain environmental changes correlate with increases in pulmonary condition severity.

In step 1360, a deviation of the current severity metric from the user's baseline is used to predict pulmonary exacerbation or attack. This may involve calculating a risk score to predict the pulmonary exacerbation or attack. If needed, in step 1370, an alert is issued to the user or to one or more other devices if the risk score exceeds a specified threshold. The threshold can vary based on age, gender, comorbidities (such as a heart condition), and the like.

Once again, the method 1300 shown in FIGURE 13 could be implemented in any suitable manner, such as entirely within an electronic device 101 or using a combination of devices. For instance, an electronic device 101 could collect data and provide the data to a server 106, which could then process the data and generate any necessary alarms.

Although FIGURE 13 illustrates one example of a method 1300 of issuing an alert based on collection of pulmonary data, various changes can be made to FIGURE 13. For example, various steps in FIGURE 13 could overlap, occur in parallel, occur serially, occur in a different order, or occur any number of times.

Although this disclosure has been described with reference to various example embodiments, various changes and modifications may be suggested to one skilled in the art. The invention is defined in the appended claims.

**Claims**

**1.** A computer program adapted to perform a method for pulmonary condition monitoring, the method comprising:

obtaining motion data collected by one or more other electronic devices (101);

obtaining audio data received by a separate microphone;
identifying respiratory phases of the user's breathing based on the audio data;
producing breathing waveform data based on the motion data;
**characterised in that** the method performed by the computer program further comprises:

converting the audio data into image data;
identifying an abnormal sound associated with the user's breathing based on the image data; and
identifying a pulmonary condition of the user based on the abnormal sound, the identified respiratory phases and the breathing waveform data.

2. The computer program of Claim 1, the method further comprises synchronizing the motion data with the audio data; and
wherein the identifying respiratory phase comprises analyzing the synchronized data to identify the respiratory phases.

3. The computer program of Claim 1, the method further comprises detecting abnormal waveforms based on the breathing waveform data; and
wherein the identifying of the pulmonary condition comprises identifying the pulmonary condition of the user further based on the abnormal waveforms.

4. The computer program of Claim 1, the method further comprising:

determining a respiration phase of the user's breathing in which the abnormal sound occurs, wherein the respiration phase includes at least one of an inspiration phase or an expiration phase;
determining an inspiration phase to expiration phase ratio of the user's breathing;
determining a duration of the abnormal sound; and
determining an intensity of the abnormal sound.

5. The computer program of Claim 4, the method further comprises determining severity of the pulmonary condition based on at least one from among the respiratory phase, the abnormal sound, the inspiration to expiration ratio, the duration of the abnormal sound and the intensity of the abnormal sound.

6. An electronic device (101) comprising:

at least one memory (130); and
at least one processor (120) coupled to the at least one memory, the at least one processor configured to:

obtain motion data collected by one or more other electronic devices;
obtain audio data received by a separate microphone;
identify respiratory phases of the user's breathing based on the audio data;
produce breathing waveform data based on the motion data;

**characterised in that** the processor is further configured to:

convert the audio data into image data;
identify an abnormal sound associated with the user's breathing based on the image data; and
identify a pulmonary condition of the user based on the abnormal sound, the identified respiratory phases and the breathing waveform data.

7. The electronic device (101) of Claim 6,
wherein the at least one processor (120) is further configured to synchronize the motion data with the audio data.

8. The electronic device (101) of Claim 6,
wherein the at least one processor (120) is further configured to:

detect abnormal waveforms based on the breathing waveform data; and
identify the pulmonary condition of the user based on the abnormal waveforms.

**9.** The electronic device (101) of Claim 6,
wherein the at least one processor (120) is further configured to:

determine a respiration phase of the user's breathing in which the abnormal sound occurs, wherein the respiration phase includes at least one of an inspiration phase or an expiration phase;
determine an inspiration phase to expiration phase ratio of the user's breathing;
determine a duration of the abnormal sound; and
determine an intensity of the abnormal sound.

**10.** The electronic device (101) of Claim 9,
wherein the at least one processor (120) is further configured to determine severity of the pulmonary condition based on at least one from among the respiratory phase, the abnormal sound, the inspiration to expiration ratio, the duration of the abnormal sound and the intensity of the abnormal sound.

**11.** A computer readable medium containing a program of any one of claims 1 to 5.

**Patentansprüche**

**1.** Computerprogramm, das angepasst ist, um ein Verfahren zum Überwachen von Lungenzustand auszuführen, das Verfahren umfassend:

Erlangen von Bewegungsdaten, die von einem oder mehreren anderen elektronischen Vorrichtungen (101) gesammelt werden;
Erlangen von Audiodaten, die von einem separaten Mikrofon empfangen werden;
Identifizieren von respiratorischen Phasen der Atmung des Benutzers basierend auf den Audiodaten;
Erzeugen von Atmungswellenformdaten basierend auf den Bewegungsdaten;
**dadurch gekennzeichnet, dass** das durch das Computerprogramm ausgeführte Verfahren ferner Folgendes umfasst:

Konvertieren der Audiodaten in Bilddaten;
Identifizieren eines anormalen Geräusches, das mit der Atmung des Benutzers assoziiert ist, basierend auf den Bilddaten; und
Identifizieren eines Lungenzustands des Benutzers basierend auf dem anormalen Geräusch, den identifizierten respiratorischen Phasen und den Atmungswellenformdaten.

**2.** Computerprogramm nach Anspruch 1, wobei das Verfahren ferner ein Synchronisieren der Bewegungsdaten mit den Audiodaten umfasst; und
wobei das Identifizieren von respiratorischer Phase ein Analysieren der synchronisierten Daten umfasst, um die respiratorischen Phasen zu identifizieren.

**3.** Computerprogramm nach Anspruch 1, wobei das Verfahren ferner ein Erkennen anormaler Wellenformen basierend auf den Atmungswellenformdaten umfasst; und
wobei das Identifizieren des Lungenzustands ein Identifizieren des Lungenzustands des Benutzers ferner basierend auf den anormalen Wellenformen umfasst.

**4.** Computerprogramm nach Anspruch 1, wobei das Verfahren ferner Folgendes umfasst:

Bestimmen einer respiratorischen Phase der Atmung des Benutzers, in der das anormale Geräusch auftritt, wobei die respiratorischen Phase mindestens eine von einer Einatmungsphase oder eine Ausatmungsphase beinhaltet;
Bestimmen eines Verhältnisses von Einatmungsphase zu Ausatmungsphase der Atmung des Benutzers;
Bestimmen einer Dauer des anormalen Geräuschs; und
Bestimmen einer Intensität des anormalen Geräuschs.

**5.** Computerprogramm nach Anspruch 4, wobei das Verfahren ferner ein Bestimmen des Schweregrads des Lungenzustands basierend auf mindestens einem von der respiratorischen Phase, dem anormalen Geräusch, dem Verhältnis von Einatmung zu Ausatmung, der Dauer des anormalen Geräuschs und der Intensität des anormalen Geräuschs umfasst.

**6.** Elektronische Vorrichtung (101) umfassend:

mindestens einen Speicher (130); und
mindestens einen Prozessor (120), der mit dem mindestens einen Speicher gekoppelt ist, wobei der mindestens eine Prozessor zu Folgendem konfiguriert ist:

Erlangen von Bewegungsdaten, die von einem oder mehreren anderen elektronischen Vorrichtungen gesammelt werden;
Erlangen von Audiodaten, die von einem separaten Mikrofon empfangen werden;
Identifizieren von respiratorischen Phasen der Atmung des Benutzers basierend auf den Audiodaten;
Erzeugen von Atmungswellenformdaten basierend auf den Bewegungsdaten;

**dadurch gekennzeichnet, dass** der Prozessor ferner zu Folgendem konfiguriert ist:

Konvertieren der Audiodaten in Bilddaten;
Identifizieren eines anormalen Geräusches, das mit der Atmung des Benutzers assoziiert ist, basierend auf den Bilddaten; und
Identifizieren eines Lungenzustands des Benutzers basierend auf dem anormalen Geräusch, den identifizierten respiratorischen Phasen und den Atmungswellenformdaten.

**7.** Elektronische Vorrichtung (101) nach Anspruch 6,
wobei der mindestens eine Prozessor (120) ferner konfiguriert ist, um die Bewegungsdaten mit den Audiodaten zu synchronisieren.

**8.** Elektronische Vorrichtung (101) nach Anspruch 6,
wobei der mindestens eine Prozessor (120) ferner zu Folgendem konfiguriert ist:

Erkennen anormaler Wellenformen basierend auf den Atmungswellenformdaten; und
Identifizieren des Lungenzustands des Benutzers basierend auf den anormalen Wellenformen.

**9.** Elektronische Vorrichtung (101) nach Anspruch 6,
wobei der mindestens eine Prozessor (120) ferner zu Folgendem konfiguriert ist:

Bestimmen einer respiratorischen Phase der Atmung des Benutzers, in der das anormale Geräusch auftritt, wobei die respiratorischen Phase mindestens eine Einatmungsphase oder eine Ausatmungsphase beinhaltet;
Bestimmen eines Verhältnisses von Einatmungsphase zu Ausatmungsphase der Atmung des Benutzers;
Bestimmen einer Dauer des anormalen Geräuschs; und
Bestimmen einer Intensität des anormalen Geräuschs.

**10.** Elektronische Vorrichtung (101) nach Anspruch 9,
wobei der mindestens eine Prozessor (120) ferner konfiguriert ist, um einen Schweregrad des Lungenzustands basierend auf mindestens einem von der respiratorischen Phase, dem anormalen Geräusch, dem Verhältnis von Einatmung zu Ausatmung, der Dauer des anormalen Geräuschs und der Intensität des anormalen Geräuschs zu bestimmen.

**11.** Computerlesbares Medium, das ein Programm nach einem der Ansprüche 1 bis 5 enthält.

**Revendications**

**1.** Programme informatique adapté à l'exécution d'un procédé de surveillance d'état pulmonaire, le procédé comprenant :

l'obtention de données de mouvement collectées par un ou plusieurs autres dispositifs électroniques (101) ;
l'obtention de données audio reçues par un microphone distinct ;
l'identification des phases respiratoires de la respiration de l'utilisateur en fonction des données audio ;

la production de données de formes d'ondes respiratoires en fonction des données de mouvement ;
**caractérisé en ce que** le procédé exécuté par le programme informatique comprend en outre :

la conversion des données audio en données d'images ;
l'identification d'un son anormal associé à la respiration de l'utilisateur en fonction des données d'images ; et

l'identification d'un état pulmonaire de l'utilisateur en fonction du son anormal, des phases respiratoires identifiées et des données de formes d'ondes respiratoires.

2. Programme informatique selon la revendication 1, le procédé comprenant en outre la synchronisation des données de mouvement avec les données audio ; et
ladite phase respiratoire de l'identification comprenant l'analyse des données synchronisées de manière à identifier les phases respiratoires.

3. Programme informatique selon la revendication 1, le procédé comprenant en outre la détection des formes d'ondes anormales en fonction des données de formes d'ondes respiratoires ; et
ladite identification de l'état pulmonaire comprenant l'identification de l'état pulmonaire de l'utilisateur en fonction en outre des formes d'ondes anormales.

4. Programme informatique selon la revendication 1, le procédé comprenant en outre :

la détermination d'une phase respiratoire de la respiration de l'utilisateur dans laquelle le son anormal se produit, ladite phase respiratoire comprenant au moins une parmi une phase d'inspiration ou une phase d'expiration ;
la détermination d'un rapport de la phase d'inspiration à la phase d'expiration de la respiration de l'utilisateur ;
la détermination de la durée du son anormal ; et
la détermination de l'intensité du son anormal.

5. Programme informatique selon la revendication 4, le procédé comprenant en outre la détermination de la gravité de l'état pulmonaire en fonction d'au moins un parmi la phase respiratoire, le son anormal, le rapport de l'inspiration à l'expiration, la durée du son anormal et l'intensité du son anormal.

6. Dispositif électronique (101) comprenant :

au moins une mémoire (130) ; et
au moins un processeur (120) couplé à ladite au moins une mémoire, ledit au moins un processeur étant configuré pour :

obtenir des données de mouvement collectées par un ou plusieurs autres appareils électroniques ;
obtenir des données audio reçues par un microphone distinct ;
identifier les phases respiratoires de la respiration de l'utilisateur en fonction des données audio ;
produire des données de formes d'ondes respiratoires en fonction des données de mouvement ;

**caractérisé en ce que** le processeur est en outre configuré pour :

convertir les données audio en données d'images ;
identifier un son anormal associé à la respiration de l'utilisateur en fonction des données d'images ; et
identifier un état pulmonaire de l'utilisateur en fonction du son anormal, des phases respiratoires identifiées et des données de formes d'ondes respiratoires.

7. Dispositif électronique (101) selon la revendication 6,
ledit au moins un processeur (120) étant en outre configuré pour synchroniser les données de mouvement avec les données audio.

8. Dispositif électronique (101) selon la revendication 6,
ledit au moins un processeur (120) étant en outre configuré pour :

détecter les formes d'ondes anormales en fonction des données de formes d'ondes respiratoires ; et
identifier l'état pulmonaire de l'utilisateur en fonction des formes d'ondes anormales.

**9.** Dispositif électronique (101) selon la revendication 6,
ledit au moins un processeur (120) étant en outre configuré pour :

déterminer une phase respiratoire de la respiration de l'utilisateur dans laquelle le son anormal se produit, ladite phase respiratoire comprenant au moins une parmi une phase d'inspiration ou une phase d'expiration ;
déterminer le rapport de la phase d'inspiration à la phase d'expiration de la respiration de l'utilisateur ;
déterminer la durée du son anormal ; et
déterminer l'intensité du son anormal.

**10.** Dispositif électronique (101) selon la revendication 9,
ledit au moins un processeur (120) étant en outre configuré pour déterminer la gravité de l'état pulmonaire en fonction d'au moins un parmi la phase respiratoire, le son anormal, le rapport de l'inspiration à l'expiration, la durée du son anormal et l'intensité du son anormal.

**11.** Support lisible par ordinateur contenant un programme selon l'une quelconque des revendications 1 à 5.

FIGURE 1

FIGURE 2

FIGURE 3

EP 3 868 293 B1

FIGURE 4A

FIGURE 4B

FIGURE 5A

FIGURE 5B

FIGURE 5C

FIGURE 6

FIGURE 7

FIGURE 8

FIGURE 9

EP 3 868 293 B1

FIGURE 10

FIGURE 11

1210

Collect Feedback:
Refute / Confirm

1220

Determine
Baseline

1240

Predict
Exacerbation

External Triggers

1230

# FIGURE 12

```
                ┌─────────────────────────────────────┐
                │            Collect Data              │────── 1310
                └─────────────────────────────────────┘
                                   │
                                   ▼
                ┌─────────────────────────────────────┐
                │  Detect adventitous sound, respiratory phase │────── 1320
                │         and relevant features        │
                └─────────────────────────────────────┘
                                   │
                                   ▼
                ┌─────────────────────────────────────┐
                │        Determine severity level      │────── 1330
                └─────────────────────────────────────┘
                                   │
```

┌───────────────────────────────┐              ┌───────────────────────────┐
│      Use severity metric       │              │   Determine potential     │
│   time series to construct     │── 1340       │  triggers of pulmonary    │
│      baseline severity         │              │    exacerbations from     │
│        condition               │   1350 ──    │  severity metric time     │
│                                │              │        series             │
└───────────────────────────────┘              └───────────────────────────┘

```
                ┌─────────────────────────────────────┐
                │  Determine deviation of severity metric from │────── 1360
                │  baseline to predict pulmonary exacerbation  │
                └─────────────────────────────────────┘
                                   │
                                   ▼
                ┌─────────────────────────────────────┐
                │           Generate alert             │────── 1370
                └─────────────────────────────────────┘
```

## FIGURE 13

**EP 3 868 293 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3282950 A1
- EP 3282950 A
- US 9610042 B1
- US 2015313484 A1